# EUROPEAN PATENT SPECIFICATION

(11) **EP 4 264 191 B1**
(45) Date of publication and mention of the grant of the patent: **06.11.2024**
(21) Application number: 21848251.1
(22) Date of filing: 16.12.2021
(51) Int. Cl.: G01D 11/24, G01D 4/00, G01D 21/00, G01D 21/02, G08B 21/00, G06Q 50/06, G01F 9/00, G06Q 50/163, G08B 21/20, H04L 12/28

(54) **AN APPARATUS FOR CONTROLLING WATER CONSUMPTION IN BATHROOMS**
VORRICHTUNG ZUR STEUERUNG DES WASSERVERBRAUCHS IN BADEZIMMERN
APPAREIL DE RÉGULATION DE CONSOMMATION D'EAU EN SALLES DE BAINS

(30) Priority: 18.12.2020 DK PA202070854
(43) Date of publication of application: 25.10.2023
(73) Proprietor: Aguardio ApS, 8260 Viby J (DK)
(72) Inventor: MUNCH-LAURSEN, Thomas, 8600 Silkeborg (DK); SKOUBOE, Kristian Reesen, 24944 Flensburg (DE)
(74) Representative: Kanved, Nicolai
(86) International application number: PCT/DK2021/050370
(87) International publication number: WO 2022/128036

(56) References cited:
- EP-A1- 3 457 227

## Description

### FIELD OF THE INVENTION

The present invention relates to the field of devices and systems for reducing or controlling water consumption in bathrooms. In particular, the invention relies on intelligent use of sensor technology for detecting human activity in bathrooms.

### BACKGROUND OF THE INVENTION

Water consumption and excessive use of fresh water in bathrooms have been subject to attention for decades. Fresh water shortage and increased wastewater emissions are some of the concerns that become more and more relevant as human water consumption is rapidly increasing. Usually, showers are supplied by fresh drinking water which is generally of public concern in terms of maintaining a sustainable supply of drinking water.

Additionally, showers are one of the single most water consuming installations in bathrooms, and the water often requires heavy energy consumption due to increased temperature of the water used. In terms of obtaining a sustainable level of CO2 emission, it is generally a desire to limit human activity in showers and other water consuming locations in bathrooms. Reducing water consumption in showers by minutes or even seconds would generally accomplish a milestone in energy consumption balance budgets.

Further problems with respect to water consumption arise for installations in bathrooms over time. Old installations or maintenance deficiencies often give rise to excessive use of fresh water in bathrooms. This may for example occur for running toilets where fresh drinking water may be constantly supplied and washed out due to problems with faulty flappers or broken valves. Likewise, water taps or showers may be subject to running water due to maintenance issues, but also if the installations are not closed properly or even left running after use. A fatal consequence may be floating due to running water which may have huge economic consequences. Accordingly, solutions to the problems mentioned above are highly desired and would contribute with a positive carbon footprint as well as more controlled water consumption in bathrooms. While solutions have been provided to reduce water consumption for individual installations in the prior art, for instance by applying a timer to a shower, these solutions have drawbacks since they do not allow for flexibility, human interaction, and human awareness.

Consolidated reports have shown that human awareness is important in order to address the problem of water consumption in bathrooms. Hence, there is a need for solutions that both contribute to limiting water consumption in bathrooms and at the same time provide human awareness that serves improved water utilization in the long run. Behavioral interaction data intelligently collected and further processed may contribute to increased awareness and sophisticated solutions for reducing and controlling water consumption in bathrooms.

Additionally, there is a need for solutions that in some embodiments incorporate more than a single water installation in bathrooms, contributing to a coordinated effort in controlling more than one water installation in a bathroom, such as all installations in a bathroom at the same time. Coordinated data collection from more than one water installation in a bathroom may serve to accomplish intelligent use of water installations and may contribute to identifying derived symptoms among user of the bathroom, including diseases such as incontinence and cystitis.

EP 3 457 227 A1 also relates to intelligent bathroom devices.

Finally, there is a need for solutions that can detect running water and provide alerts when needed under certain conditions, as well as solutions that may help nudge humans to use bathroom facilities in the most proper way, including coordinated use of water installations for hygiene purposes. These needs and problems in addition to further problems may be solved according to the present invention described in the following description and outlined in detail in the accompanying drawings.

### SUMMARY OF THE INVENTION

The invention is defined by the appended claims. According to claim 1, there is provided an apparatus (100) for reducing or controlling water consumption in bathrooms, the apparatus (100) comprising a housing (105) and being configured to receive data from one or more water consumption locations in a bathroom, the housing (105) comprising:
one or more sensors (107) including a motion sensor (110) configured to detect a movement in a certain distance from the apparatus in a bathroom as a first indication of activity in the bathroom, an audio sensor (115) configured to monitor a sound of running water from one or more water consumption locations in a bathroom as a second indication of activity in the bathroom, optionally a humidity sensor (120) configured to monitor the ambient humidity in a bathroom as a third indication of activity in the bathroom, and optionally an ambient temperature sensor (200);
a controller (125) configured to qualify at least a part of the data received from the one or more sensors (107) by means of an algorithm to obtain qualified data, wherein the controller (125) is configured to qualify at least a part of the data received from the audio sensor (115) as qualified audio data by means of an algorithm based on a reference audio standard;
a transmitting member (130) configured to transmit the qualified data at a given point of time to an external unit (135), optionally via a mobile device (230), by means of low power signals; and
a unique identification means (140) being readable or scannable by a mobile device and configured to include output data based on the qualified data for reducing or controlling water consumption in the bathroom.

According to claim 10, there is provided a system (201) for reducing or controlling water consumption in bathrooms, the system comprising:
an apparatus (100) according to any one of the preceding claims;
an external unit (135) configured to receive qualified data from the apparatus (100) at a given point of time, optionally via a mobile device (230), by means of low power signals, and being configured to process data based on the qualified data and/or based on further qualification of data, such as humidity data, and configured to incorporate output data in a unique identification means (140) being readable by a mobile device (230) on the apparatus (100) for reducing or controlling water consumption in a bathroom;
optionally a commands receiving member (240) in the apparatus (100) configured to receive commands based on the qualified data at a given point of time from the external unit (135) by means of low power signals, and the controller (125) being configured to integrate the commands; and
a mobile device (230) operable to read the unique identification means (140).

According to claim 14, there is provided a method of reducing or controlling water consumption in bathrooms, the method comprising the steps of:
providing an apparatus (100) being configured to receive data from one or more water consumption locations in a bathroom, the apparatus comprising a housing (105) including one or more sensors (107), a controller (125), a transmitting member (130), and a unique identification means (140);
detecting a movement in a certain distance from the apparatus (100) in a bathroom as a first indication of activity in the bathroom by means of a motion sensor (110), detecting a sound of running water from one or more water consumption locations in a bathroom as a second indication of activity in the bathroom by means of an audio sensor (115), optionally detecting the ambient humidity in a bathroom as a third indication of activity in the bathroom by means of a humidity sensor (120), and optionally detecting ambient temperature by means of an ambient temperature sensor (200);
qualifying at least a part of the data received from the one or more sensors (107) by means of an algorithm to obtain qualified data, wherein the data includes data received from the audio sensor (115) as qualified audio data based on a reference audio standard;
transmitting the qualified data at a given point of time to an external unit (135), optionally via a mobile device (230), by means of low power signals; and
subjecting output data based on the qualified data for reducing or controlling water consumption in the bathroom to a unique identification means (140) being readable or scannable by a mobile device (230).

One of the advantages of the present invention is that water consumption may be reduced for water installations in bathroom, such as showers, by minutes or even seconds. Reducing water consumption in showers by minutes or even seconds would generally accomplish a milestone in energy consumption balance budgets.

Generally, use of sensors for detection of human presence and interaction in bathrooms is difficult as it requires recognition of multiple variables in order to identify and qualify such presence and interaction, particularly if an intelligent solution is to be established for behavioral interaction.

According to the invention, there is provided one or more sensors (107) including a motion sensor (110) configured to detect a movement in a certain distance from the apparatus in a bathroom as a first indication of activity in the bathroom, and an audio sensor (115) configured to monitor a sound of running water from one or more water consumption locations in a bathroom as a second indication of activity in the bathroom.

In the present context "as a first indication of activity in the bathroom" is intended to mean a first sequence of identification means, and "as a second indication of activity in the bathroom" is intended to mean a second sequence of identification means. Hence, the apparatus of the invention detects motion as the first activity and audio as the next activity. However, as time continues both sensors may be in action with an overlap and may end detecting at different points of time. These precise calculations of activity are incorporated in an algorithm and/or preset in the controller.

As a third step, a humidity sensor configured to monitor the ambient humidity in a bathroom may optionally be applied as a third indication of activity in the bathroom. In some embodiments, this third option is preferred, such as in the case where data from the first two sensors is to be validated as properly reflecting water consumption at a location in the bathroom. Also, this third sensor may be used to detect climate conditions in a bathroom, preferably in connection with an optional ambient temperature sensor. This may prevent formation of mold in the bathroom or other derived problems to be monitored.

Also, for the optional third humidity sensor, the motion sensor and audio sensor may continue detecting, and preferably do so. In the present context "as a third indication of activity in the bathroom" is intended to mean a third sequence of identification means. Hence, the apparatus of the invention optionally detects humidity as the third activity, while still detecting motion and/or audio activity. However, as time continues all sensors may be in action with an overlap and may end detecting at different points of time. These precise calculations of activity is incorporated in an algorithm or preset in the controller.

According to the invention, the housing of the apparatus comprises a controller (125) configured to qualify at least a part of the data received from the one or more sensors (107) by means of an algorithm to obtain qualified data. This controller may be a CPU processor or other means available to perform qualification of data by means of an algorithm. The housing may also include storage means, such as a memory card that allows data collection for a longer period of time before further transmission. Further submission may be activated when needed or at predetermined time intervals, such as one time a day, two times a day, each two hours, or the like. It may also be real time transmission. The controller receives signals from the sensoring means and may do so directly, or through other components in the apparatus.

The term "qualify" is intended to mean quality controlling of real data for human interaction by means of an algorithm, such as iteration or numerical analysis of data received. An algorithm may not be needed for all the sensors involved in data collection, but may be used for only one sensoring means, such as the audio sensor, two or more sensoring means. Noise from other sources may intervene with noise from for instance a shower and may preferably be discarded. In this instance, the algorithm is configured to fractionate the sound of running water from background noise and even sources with very similar frequency as running water, such as a hair dryer. The controller may provide a first set of qualified data which later may be supplemented with further qualification, for instance in an external unit. Accordingly, the term "qualified" in the present context is intended to mean motion data and audio data that involves human interaction, optionally humidity data that involves human interaction.

Generally, the apparatus and system is set up to estimate water consumption as a function of time, e.g., the total time of each shower or incidences of running water of a certain time length. One option is to have the display in the apparatus visualizing the water consumption time for a given water installation, such as a shower. In this case, real time data may be available for inspection directly in addition to data transmitted to an external unit. These data may be qualified by the controller before they are visualized on the display. In the alternative, these data visualized on the display may be raw data that has not undergone any qualification process.

According to the invention, a transmitting member (130), such as an antenna, configured to transmit the qualified data at a given point of time to an external unit (135) is provided in the housing of the apparatus which uses low power signals. Optionally the qualified data may also be processed via a mobile device (230) by means of low power signals to an external unit. In the present context the "external unit" may be a computing device or simply an accessible cloud storage solution, optionally further comprising a dashboard for tracking and monitoring one or more bathrooms, such as bathrooms in a building, hotel or building complex. Further qualification of data may be provided by the external unit, such as confirmation of audio and motion by humidity data which may be somehow delayed due to a relatively slow increase in humidity upon water installation usage. This option may serve to be a further quality check of data. Further qualification may preferably be made externally but may also be conducted in the controller in a pre-setup.

In the present context a "bathroom" should be understood in a broad sense. While showers may usually be located in rooms dedicated for the purpose, water taps or toilets may be located in rooms that are not particularly dedicated for the purpose. These rooms include hotel rooms with a wash and a water tap located in a main room or other rooms that are not addressed for the purpose. Also, bathrooms may be understood as rooms dedicated for toilets and water taps, and for rooms only dedicated for showers. Additionally, shower rooms in public places such as swimming facilities fall under the definition of "bathroom" in the present context. A person skilled in the art would provide a reasonable interpretation in view of the present invention.

In the present context, when "low power signals" is mentioned, this may include narrow band low power signals or other low power signals, such as Bluetooth. In the present context, Wi-Fi is not intended for the present invention as the battery requirements are too high. Use of low power signals were seen by the inventors to be a huge advantage according to the invention, which may allow the apparatus to be placed in locations without constant wired energy supply, even in remote places hardly accessible for maintenance.

Communication from the apparatus to the external unit is preferably made with narrow band low power signals. Communication via a mobile device to the external apparatus is preferably made with Bluetooth but does not necessarily need to be so but can also be with other low power signals. The use of low power signals in the present invention was not expected by the inventors to give this much added benefit that was seen in the resulting product. Accordingly, energy consuming Wi-Fi may be avoided and frequent charging of batteries may be avoided. Also, the system may be particularly vulnerable by using more energy consuming sources due to the relative humid conditions in bathrooms, and since the apparatus may be susceptible to direct water exposure from water installations.

In some embodiments of the invention, the unique identification means (140) is a matrix barcode, such as a quick response (QR) code, or a near-field communication (NFC) chip. Other means are also within the scope of the invention. In some embodiments, a quick response (QR) code is preferred. The matrix barcode may be visible on the housing but may in a preferred embodiment be readable or scannable by a mobile device and located on a display under a transparent portion of the cover of the housing.

In the present context, when the apparatus is mentioned to comprise a unique identification means, such as a QR code, it may we visible and readable at any given point and time but may also be visible and readable for only a period of time. For instance, the display of the present invention may show the clock and/or water consumption time, and when needed also the QR code. An NFC chip, if present, may be scannable at any given point of time and may be hidden below the cover.

The great advantage of using identification means in the present invention was a surprise to the inventors. It was not expected that for instance a QR code was even possible to apply in the apparatus and would be possible to apply without compromising the function of the apparatus. Also, the QR code provides for a very flexible solution and may be scannable or readable on the apparatus directly by a user of the water installation in the bathroom, without using an app. The output data based on qualified data may be directly presented to the user and applied directly in the bathroom.

The unique code makes it possible to apply a central unit, the external unit, that may collect qualified data from a number of devices and allocate the specific qualified data for the specific bathroom when needed. Hence, the system according to the invention and the apparatus as such, provide a huge benefit compared to prior art systems, where user interaction directly in a bathroom is usually not provided for.

In some embodiments of the invention, the controller (125) is configured to qualify at least a part of the data received from the motion sensor (110) as qualified motion data by means of an algorithm based on a reference motion standard. In this embodiment, the motion sensor may qualify data by distance to a moving object, or it may use artificial intelligence to detect a moving object.

In the invention, the controller (125) is configured to qualify at least a part of the data received from the audio sensor (115) as qualified audio data by means of an algorithm based on a reference audio standard. Noise from the surroundings may be filtered in order to detect running water. Since the noise of running water from various showers may be different, qualification may also take this aspect into account.

Also, noise that has a similar characteristics to running water may be filtered and qualification may take this into account as well. For instance, noise from a hair dryer was seen by the inventors to cause huge problems in differentiation from the noise of running water. Qualification of data in this respect is also accounted for as well as other external noise sources. It was a surprise to the inventors that it was possible in the end to use an audio sensor to detect running water due to the difficulties in filtering noise from other sources.

In some embodiments of the invention, the controller (125) is configured to both qualify at least a part of the data received from the motion sensor (110) as qualified motion data by means of an algorithm based on a reference motion standard and is configured to qualify at least a part of the data received from the audio sensor (115) as qualified audio data by means of an algorithm based on a reference audio standard In some embodiments of the invention, the transmitting member (130) is configured to transmit motion data and audio data at a given point of time if the motion data and the audio data are qualified by the controller (125). In this embodiment, data is not transmitted if both motion data and audio data is disqualified, which may be the case when human interaction is calculated not to be present.

In some embodiments of the invention, the transmitting member (130) is configured to transmit humidity data at a given point of time if the humidity data is qualified by the controller (125).

In some embodiments of the invention, the transmitting member (130) is configured to transmit humidity data at a given point of time if the humidity data is qualified by the controller (125). In this embodiment, humidity data is not transmitted if both are disqualified, which may be the case when human interaction is calculated not to be present.

In some embodiments of the invention, the identification means (140) is configured to include output data if motion data and audio data at a given point of time are qualified by the controller (125), and humidity data is qualified by the controller (125) or the external unit (135) to encompass a predetermined change in humidity as the third indication of activity in the bathroom.

In some embodiments of the invention, the transmitting member (130) is configured to transmit motion data, audio data and humidity data at a given point of time if the motion data, the audio data, and the humidity data are qualified by the controller (125).

In some embodiments of the invention, the one or more sensors (107) includes an ambient temperature sensor (200).

In some embodiments of the invention, the transmitting member (130) is configured to transmit temperature data at a given point of time if the temperature data is qualified by the controller (125).

In some embodiments of the invention, the identification means (140) is configured to include output data if motion data and audio data at a given point of time are qualified by the controller (125), humidity data is qualified by the controller (125) or the external unit (135) to encompass a predetermined change in humidity as the third indication of activity in the bathroom, and temperature data is qualified by the controller (125) or the external unit (135).

In some embodiments of the invention, the transmitting member (130) is configured to transmit motion data, audio data, humidity data and temperature data at a given point of time if the motion data, the audio data, the humidity data, and the temperature data are qualified by the controller (125).

In some embodiments of the invention, the motion sensor (110) is a passive infrared sensor (PIR sensor) located below the level of the surface of the housing. Since privacy is of outmost importance in bathroom, the inventors of the present invention developed means for hiding the motion sensor. The solution in this embodiment is highly beneficial and unexpectedly provides for accurate motion detection, even though the sensor is not as exposed as in prior art solutions.

In some embodiments of the invention, data are integrated by the controller (125) or the external unit (135).

In some embodiments of the invention, motion data, audio data, and humidity data are integrated by the controller (125) or the external unit (135).

In some embodiments of the invention, motion data, audio data, humidity data and temperature data are integrated by the controller (125) or the external unit (135).

In some embodiments of the invention, the one or more sensors (107) includes a carbon dioxide sensor.

In some embodiments of the invention, the transmitting member (130) is configured to transmit carbon dioxide data at a given point of time if the carbon dioxide data is qualified by the controller (125).

In some embodiments of the invention, the identification means (140) is configured to include output data if carbon dioxide data at a given point of time is qualified by the controller (125) or the external unit (135).

In some embodiments of the invention, the transmitting member (130) is configured to transmit motion data, audio data, humidity data, temperature data and carbon dioxide data at a given point of time if the motion data, the audio data, the humidity data, the temperature data, and the carbon dioxide data are qualified by the controller (125).

In some embodiments of the invention, the one or more water consumption locations in a bathroom comprises the space defined by the inside of the walls of the bathroom, excluding locations within or outside the walls of the bathroom.

In some embodiments of the invention, the housing (105) further comprises a commands receiving member (145) configured to receive commands based on the qualified data at a given point of time from the external unit (135) by means of low power signals, and the controller (125) is configured to integrate the commands. In this embodiment, the controller may be upgraded to perform better based on qualification of data, preferably based on a huge set of data, such as metadata. In the present context "metadata" is intended to mean integration of data for more than one bathroom, such as for numerous bathrooms. The commands in the present context may serve to change the preset conditions in the controller or may even provide learning algorithms to the controller for better performing qualification of data.

The external unit (135) may in addition to the controller in the apparatus comprise an algorithm that may serve to improve the general function of the apparatus. For instance, the algorithm may work to improve the qualified data already received from the controller and send it back to the controller for improved performance of the apparatus. This may be by means of a numeral analysis but also artificial intelligence. Typically, such commands are received by a commands receiving member (145) in the apparatus. One example of commands may be commands for improving the apparatus' water consumption performance or ability to detect water consumption over time.

In some embodiments of the invention, the apparatus comprises a bracket (150) with a magnetic member (155), and the housing (105) comprises a hall sensor (160) to detect the magnetic member (155) upon placing the housing in the bracket (150) whereby the apparatus (100) is activated. In this embodiment, installation is made very easy for a user. Essentially, when the apparatus is mounted on the bracket, the system automatically starts up. Since the apparatus may preferably have a build-in chip for external communication, the system may automatically go online and be communicably connected with the external unit.

In some embodiments of the invention, the housing of the apparatus comprises a front cover (170) including a transparent centrally arranged portion (175) facing the front of said cover (170) that allows visibility to a display (180) positioned below the transparent centrally arranged portion (175), and a portion with a substantially thinner wall thickness (185) that allows functioning of the motion sensor (110) located below the level of the surface of the housing.

In some embodiments of the invention, the housing of the apparatus comprises a back cover (190) connectable and sealable with the front cover (170), the back cover (190) encasing a printing board (195), the printing board (195) including the display (180) operable to visualize the unique matrix barcode (140), the one and more sensors, the controller (125), and the transmitting member (130).

In some embodiments of the invention, the housing of the apparatus comprises a back plate (210) connectable and sealable with the back cover (190), the back plate and the back cover encasing a power supply member (220) for operating the apparatus.

In some embodiments of the invention, the output data scannable or readable on the apparatus includes one or more of water-saving tips, shower statistics, water tap statistics, toilet usage statistics, running water statistics and running water alerts. The output data based on the qualified data may include further statistics. Additionally, the output data may also serve to nudge a user to use water installations differently.

With respect to the second aspect of the invention, the system may comprise an external unit (135) configured to receive qualified data from the apparatus (100) at a given point of time, optionally via a mobile device (230), by means of low power signals, and being configured to process data based on the qualified data and/or based on further qualification of data, such as humidity data, and configured to incorporate output data in a unique identification means (140) being readable by a mobile device (230) on the apparatus (100) for reducing or controlling water consumption in a bathroom.

Optionally, the system may comprise a commands receiving member (240) in the apparatus (100) configured to receive commands based on the qualified data at a given point of time from the external unit (135) by means of low power signals, and the controller (125) being configured to integrate the commands.

Additionally, the system may comprise a mobile device (230) operable to read the unique identification means (140). In the present context a "mobile device" may be any handheld means for detecting the unique identification means, such as a mobile phone or a tablet.

In some embodiments of the invention, the system further includes one or more external sensors (235) communicably coupled to the apparatus (100), optionally by Bluetooth, and further including a sensor receiving member (245) in the apparatus (100) configured to receive data from the one or more external sensors (235), and the controller (125) being configured to integrate the data. The data may be further transmitted to an external unit and/or a mobile device, as is the case for the qualified data.

In some embodiments of the invention, the one or more external sensors (235) include one or more external temperature sensors mounted to a water inlet (240) of a local installation (250) in the bathroom, including one or more inlets of a toilet (255), a water tap (257) or a shower (260), and the one or more external temperature sensors being configured to detect a temperature difference of the water in the inlet (240) compared to ambient temperature over time, whereby running water may be detected. This may be done by ultrasonic means or specific temperature sensors mounted on or around water vessels close to the water installation in the bathroom.

In some embodiments of the invention, the one or more external sensors (235) include an external temperature sensor mounted to a water inlet of a first local installation in the bathroom, such as a toilet (255), in combination with an external audio sensor in proximity to a water inlet of a second local installation in the bathroom and/or an external temperature sensor mounted to the water inlet of a second local installation in the bathroom, such as a water tap (257), whereby coordinated usage of both installations may be detected.

In some embodiments of the invention, the system further includes an alert for running water to the mobile device (230) and/or to the external unit (135). In this embodiment, running water in a toilet may be detected early and transmitted to a mobile device and/or an external unit.

In some embodiments of the invention, the apparatus (100) is included in a plurality of bathrooms, and the external unit (135) is configured to process metadata based on qualified data from a plurality of bathrooms, optionally including said metadata in the unique identification means (140) and/or a central dashboard (142).

With respect to the second aspect of the invention, the method may comprise a step of detecting a movement in a certain distance from the apparatus (100) in a bathroom as a first indication of activity in the bathroom by means of a motion sensor (110), detecting a sound of running water from one or more water consumption locations in a bathroom as a second indication of activity in the bathroom by means of an audio sensor (115), optionally detecting the ambient humidity in a bathroom as a third indication of activity in the bathroom by means of a humidity sensor (120), and optionally detecting ambient temperature by means of an ambient temperature sensor (200).

In some embodiments of the invention, the one or more external sensors (235) is located within the space defined by the inside of the walls of the bathroom, excluding locations within or outside the walls of the bathroom.

Additionally, the method may comprise a step of qualifying at least a part of the data received from the one or more sensors (107) by means of an algorithm to obtain qualified data. Further, the method may comprise a step of transmitting the qualified data at a given point of time to an external unit (135), optionally via a mobile device (230), by means of low power signals. Finally, the method may comprise a step of subjecting output data based on the qualified data for reducing or controlling water consumption in the bathroom to a unique identification means (140) being readable or scannable by a mobile device (230).

It is to be understood that elements of the system and method according to the invention may be applied in combination with the apparatus according to the invention. Hence, specific elements of the method may be applied and combined with other elements of the apparatus as described herein.

In some embodiments of the invention, the audio sensor (115) is protected from water droplets in the apparatus (100) by means of a trap (270) that serves to avoid water interference without substantially reducing audio signals, the trap (270) comprising a U-shaped air channel (275) starting from the face of a bottom portion (276) of the apparatus (100) and extending towards the audio sensor (115). This trap was surprisingly seen to work and provide added benefits in the harsh humid environment of bathrooms. For example, water droplets exposed to the apparatus was seen to be trapped in the U-shaped channel of the apparatus. This embodiment makes it possible to avoid filters that are usually seen as reducing sound quality and the sound picture in general.

In some embodiments of the invention, the humidity sensor (120) is protected from water droplets in the apparatus (100) by means of a trap (270) that serves to avoid water interference without substantially reducing audio signals, the trap (270) comprising a U-shaped air channel (285) starting from the face of a bottom portion (276) of the apparatus (100) and extending towards the humidity sensor (120). This trap was surprisingly seen to work and provide added benefits in the harsh humid environment of bathrooms. For example, water droplets exposed to the apparatus was seen to be trapped in the U-shaped channel of the apparatus. This embodiment makes it possible to avoid interference with the humidity sensor.

In some embodiments of the invention, a bracket (150) of the apparatus (100) is fixed to the housing (105) by means of a connecting member (290), such as a screw, adjusted to secure that the audio sensor (115) in the housing (105) is tuned for optimized sound picture. This embodiment was seen as a very huge advantage in that the audio sensor according to the invention may need a precise positioning in order to deliver the most advantageous sound picture. The connecting member may both be used to attach the bracket to the apparatus and at the same time be used to adjust positioning of the apparatus in a correct position.

The invention will be understood in greater detail with reference to the following figures that serve to illustrate certain particular embodiments of the invention by way of example.

### BRIEF DESCRIPTION OF DRAWINGS

Fig. **1** is a perspective view of the apparatus according to the invention, illustrating the various visible parts on top of the apparatus in an unfolded position.
Fig. **2** is top view of the apparatus with the front cover unmounted, and a printing board with a display centrally arranged and positioned in the back cover.
Fig. **3** illustrates the back of the apparatus in the top section, where the back cover and the back plate mounted to the back cover, as well as various elements on the back side of the apparatus. The bottom section of the drawing illustrates the back of the apparatus, where the back cover is made transparent in order to see the printing board underneath.
Fig. **4** is a perspective view of the bracket to be mounted on a wall and the apparatus according to the invention.
Fig. **5** is a cross-sectional view of the apparatus, where the U-shaped trap is illustrated for the audio sensor in the apparatus.
Fig. **6** is a cross-sectional view of the apparatus, where the U-shaped trap is illustrated for the humidity sensor in the apparatus.
Fig. **7** is an expanded view of the apparatus, including the front cover, printing board and back cover.
Fig. **8** is an expanded view of the apparatus, including the front cover mounted with the back cover, the energy storage means (battery), back plate and the bracket.
Fig. **9** is an overview of the system according to the invention, including the apparatus according to the invention. The large, squared box illustrates the apparatus.

### DETAILED DESCRIPTION

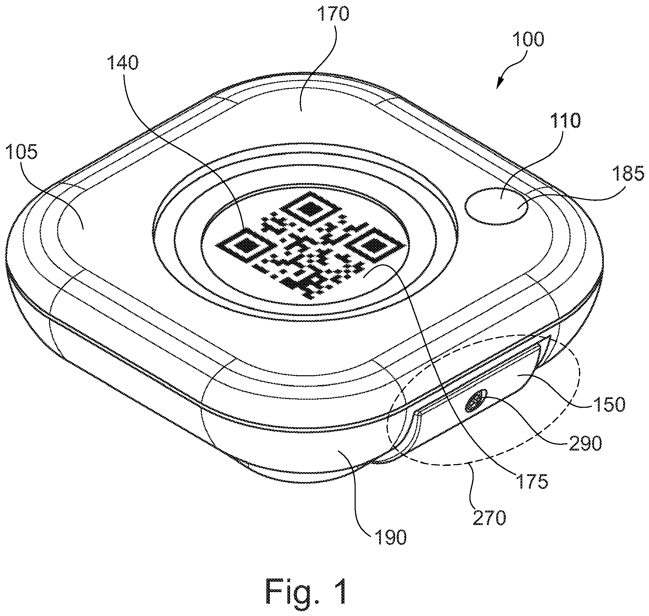

Referring now to Fig. **1****,** **2****,** **3****,** **5****,** **6****,** **7** and **8** of the drawings, there is provided an apparatus (100) for reducing or controlling water consumption in bathrooms, the apparatus (100) comprising a housing (105) and being configured to receive data from one or more water consumption locations in a bathroom. The housing (105) comprises one or more sensors (107) including a motion sensor (110) configured to detect a movement in a certain distance from the apparatus in a bathroom as a first indication of activity in the bathroom, an audio sensor (115) configured to monitor a sound of running water from one or more water consumption locations in a bathroom as a second indication of activity in the bathroom, optionally a humidity sensor (120) configured to monitor the ambient humidity in a bathroom as a third indication of activity in the bathroom, and optionally an ambient temperature sensor (200).

Further, the apparatus comprises a controller (125) configured to qualify at least a part of the data received from the one or more sensors (107) by means of an algorithm to obtain qualified data. Also, the apparatus comprises a transmitting member (130) configured to transmit the qualified data at a given point of time to an external unit (135), optionally via a mobile device (230), by means of low power signals. Finally, as illustrated the apparatus comprises a unique identification means (140) being readable or scannable by a mobile device and configured to include output data based on the qualified data for reducing or controlling water consumption in the bathroom.

Referring now to Fig. **1** of the drawings there is provided a unique identification means (140) is a matrix barcode, such as a quick response (QR) code, or a near-field communication (NFC) chip.

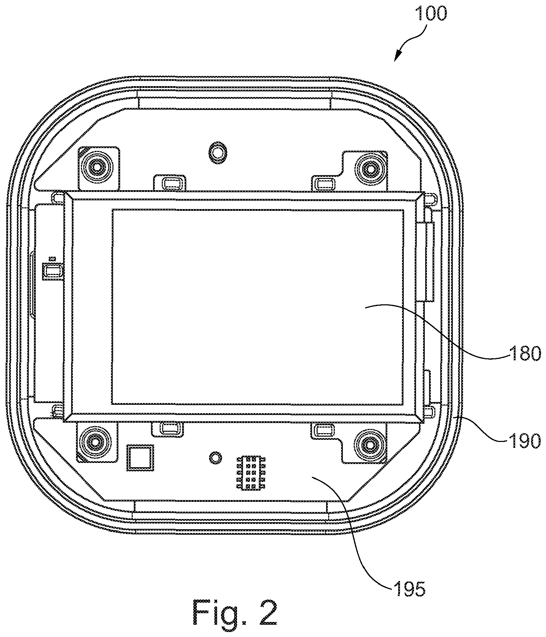

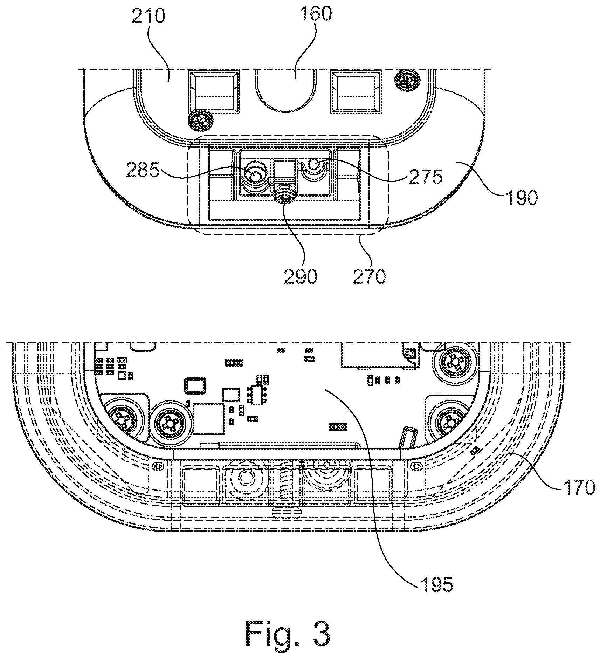

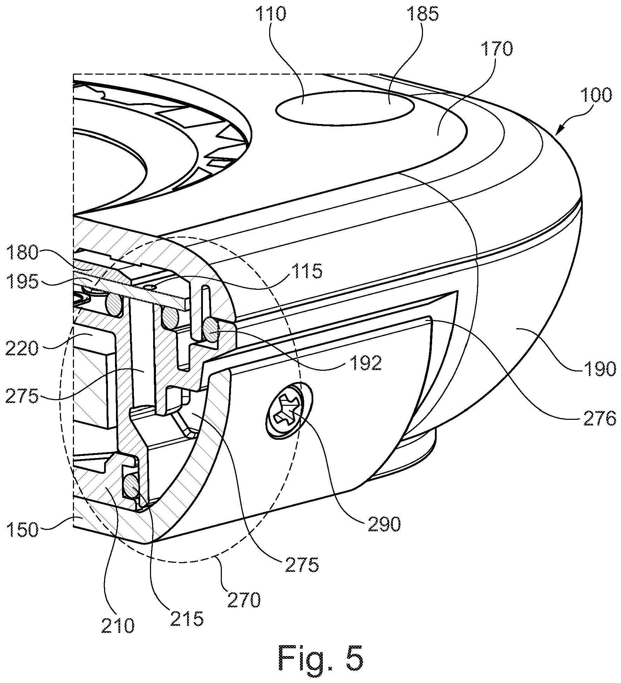

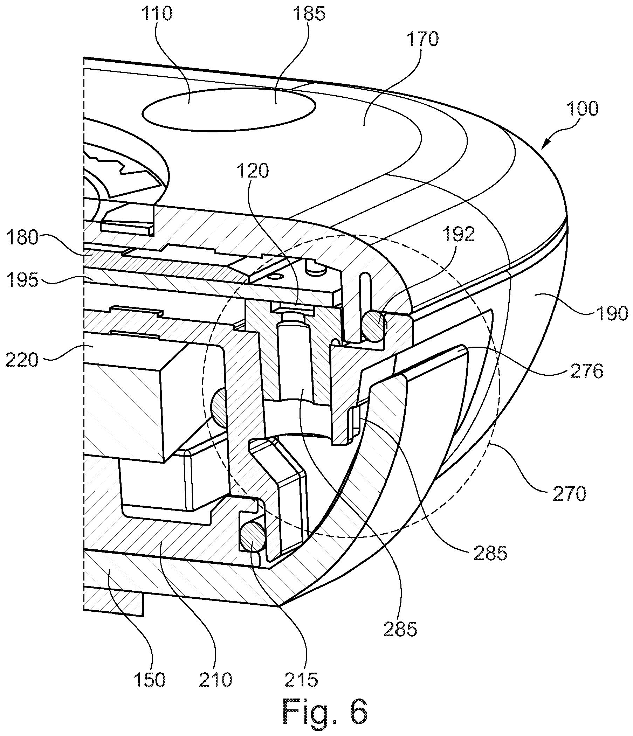

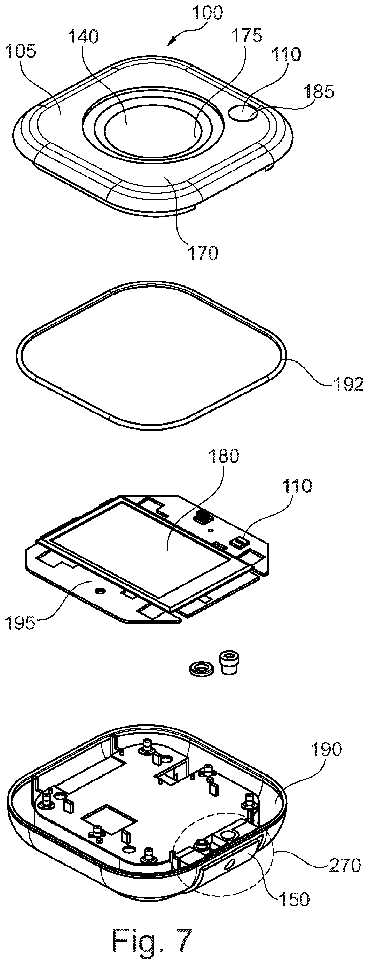

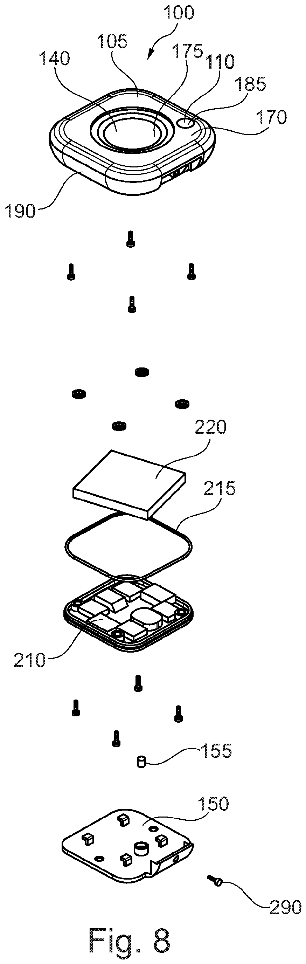

The apparatus illustrated comprises a controller (125) configured to qualify at least a part of the data received from the motion sensor (110) as qualified motion data by means of an algorithm based on a reference motion standard. The controller is located on a printing board of the apparatus, which printing board is illustrated in Fig. **2****,** **3****,** partly in Fig. **5** and **6****,** in Fig. **7** and **8****.**

The controller (125) is configured to qualify at least a part of the data received from the audio sensor (115) as qualified audio data by means of an algorithm based on a reference audio standard. Further, the controller (125) is configured to qualify at least a part of the data received from the audio sensor (115) as qualified audio data by means of an algorithm based on a reference audio standard.

The apparatus (100) illustrated comprises a transmitting member (130) configured to transmit motion data and audio data at a given point of time if the motion data and the audio data are qualified by the controller (125). The apparatus (100) illustrated is also configured to transmit humidity data at a given point of time if the humidity data is qualified by the controller (125).

With respect to the identification means (140) as illustrated in Fig. **1****,** the identification means (140) is configured to include output data if motion data and audio data at a given point of time are qualified by the controller (125), and humidity data is qualified by the controller (125) or the external unit (135) to encompass a predetermined change in humidity as the third indication of activity in the bathroom.

Fig. **1****,** **5****,** **6****,** **7** and **8** illustrates the motion sensor (110) as a passive infrared sensor (PIR sensor) located below the level of the surface of the housing. The motion sensor is a PIR as here illustrated but may also be another type of motion sensor according to the invention.

The apparatus (100) illustrated may further comprise (not illustrated here), a commands receiving member (145) configured to receive commands based on the qualified data at a given point of time from the external unit (135) by means of low power signals, and the controller (125) is configured to integrate the commands.

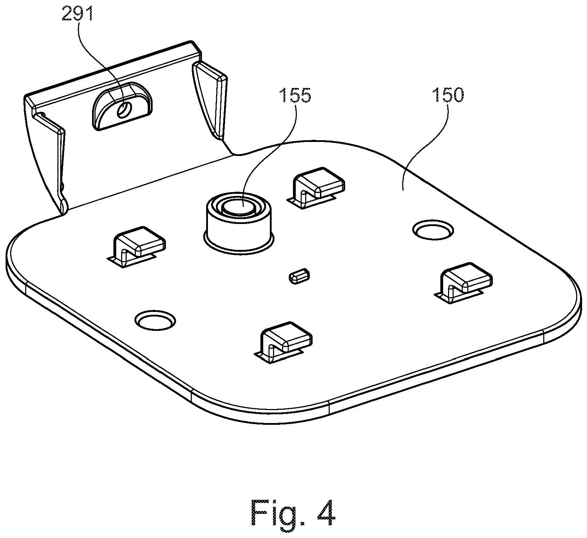

Referring now to Fig. **4****,** partly Fig. **5****,** **6** and **7****,** and Fig. **8** of the drawings, there is provided a bracket (150) with a magnetic member (155), and the housing (105) comprises a hall sensor (160) to detect the magnetic member (155) upon placing the housing in the bracket (150) whereby the apparatus (100) is activated.

Referring further to Fig. **1****,** **2****,** **3****,** **5****,** **6****,** **7** and **8** of the drawings, there is provided illustrations of the housing of the apparatus, comprising a front cover (170) including a transparent centrally arranged portion (175) facing the front of said cover (170) that allows visibility to a display (180) positioned below the transparent centrally arranged portion (175), and a portion with a substantially thinner wall thickness (185) that allows functioning of the motion sensor (110) located below the level of the surface of the housing;
a back cover (190) connectable and sealable with the front cover (170), the back cover (190) encasing a printing board (195), the printing board (195) including the display (180) operable to visualize the unique matrix barcode (140), the one and more sensors, the controller (125), and the transmitting member (130);
a back plate (210) connectable and sealable with the back cover (190), the back plate and the back cover encasing a power supply member (220) for operating the apparatus.

As illustrated in Fig. **5****,** and indicated in Fig. **1****,** **3** and **7****,** the audio sensor (115) is protected from water droplets in the apparatus (100) by means of a trap (270) that serves to avoid water interference without substantially reducing audio signals, the trap (270) comprising a U-shaped air channel (275) starting from the face of a bottom portion (276) of the apparatus (100) and extending towards the audio sensor (115).

As illustrated in Fig. **6****,** and indicated in Fig. **1****,** **3** and **7****,** the humidity sensor (120) is protected from water droplets in the apparatus (100) by means of a trap (270) that serves to avoid water interference without substantially reducing audio signals, the trap (270) comprising a U-shaped air channel (285) starting from the face of a bottom portion (276) of the apparatus (100) and extending towards the humidity sensor (120).

Furthermore, as illustrated in Fig. **1****,** **3****,** and indicated in Fig. **4** with a hole (291), **5** and **8,** there is provided a bracket (150) of the apparatus (100) fixed to the housing (105) by means of a connecting member (290), such as a screw, adjusted to secure that the audio sensor (115) in the housing (105) is tuned for optimized sound picture.

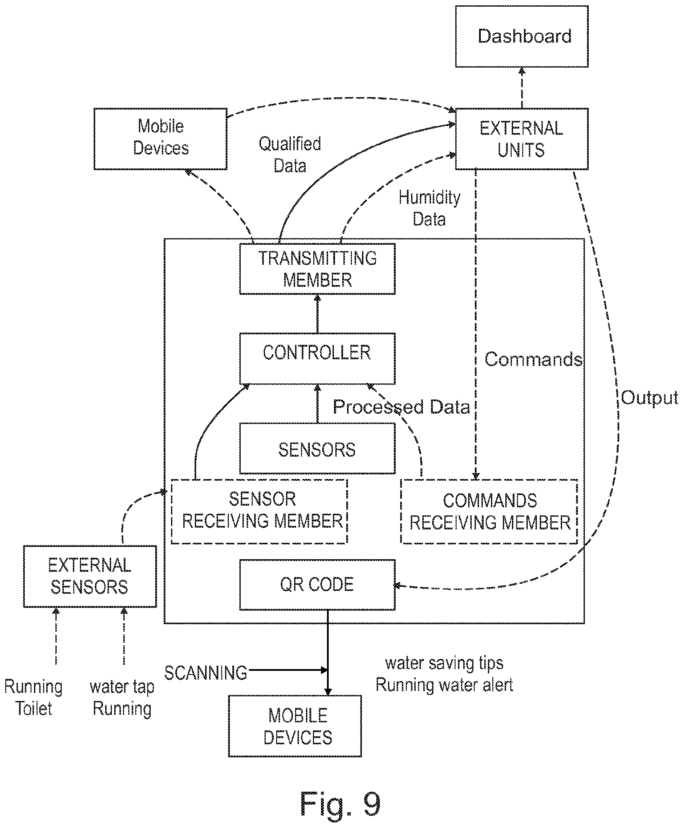

Turning to the system according to the invention, this is illustrated in Fig. 9. Here, it can also be seen how the various elements work together in the system. The system is illustrated with one or more external sensors (235) communicably coupled to the apparatus (100), optionally by Bluetooth, and further including a sensor receiving member (245) in the apparatus (100) configured to receive data from the one or more external sensors (235), and the controller (125) being configured to integrate the data.

The system may include one or more external sensors (235) include one or more external temperature sensors mounted to a water inlet (240) of a local installation (250) in the bathroom, including one or more inlets of a toilet (255), a water tap (257) or a shower (260), and the one or more external temperature sensors being configured to detect a temperature difference of the water in the inlet (240) compared to ambient temperature over time, whereby running water may be detected. Further, the system may include one or more external sensors (235) include an external temperature sensor mounted to a water inlet of a first local installation in the bathroom, such as a toilet (255), in combination with an external audio sensor in proximity to a water inlet of a second local installation in the bathroom and/or an external temperature sensor mounted to the water inlet of a second local installation in the bathroom, such as a water tap (257), whereby coordinated usage of both installations may be detected.

Further, the system may include an alert for running water to the mobile device (230) and/or to the external unit (135), such as one or more alerts (not illustrated).

The apparatus of the system may especially be adapted to be included in a plurality of bathrooms (not illustrated), and the external unit (135) is configured to process metadata based on qualified data from a plurality of bathrooms, optionally including said metadata in the unique identification means (140) and/or a central dashboard (142).

Turning to Fig. 9, the system according to the invention is illustrated and also provides an overview of the method according to the invention. The large, squared box illustrates the apparatus and the elements described in detail in the previous sections. The sensors in the apparatus include an audio sensor and a motion sensor, and optionally a humidity sensor and/or a temperature sensor. Data from the sensors in the apparatus are delivered to the controller where qualification of the data is performed. Data may further be stored in storing means in the apparatus until they are transmitted from the apparatus to an external unit, optionally via a mobile device by low power means, such as Bluetooth. In the presently preferred set-up, qualified data from the controller, optionally via other elements in the apparatus, is transmitted to an external unit by use of narrow band low power signals. Humidity data may also be transmitted to the external unit and be transmitted independently from motion and audio data.

Once, the qualified data is received by the external unit, further qualification may be performed by the external unit in order to achieve output data with high quality. In some embodiments of the invention, data from a number of bathrooms may be transmitted to the external unit and further conveyed to a dashboard where full control of various bathrooms may be performed. The high-quality output is then optionally further categorised or optimized.

If necessary, or as part of optimization procedures, commands may be transmitted back to a commands receiving member in the apparatus in order to optimize the system or pre-set requirements in the controller. For instance, more intelligent algorithms may be provided based on complete data from various bathrooms. Metadata may then be used for optimization of the systems. It may also be small adjustments or reference data that may be transmitted to the controller and incorporated in the apparatus.

Finally, output data may be directly scanned by a mobile device on the apparatus and may provide output data includes one or more of water-saving tips, shower statistics, water tap statistics, toilet usage statistics, running water statistics and running water alerts.

As a separate option that may be integrated in the system is external sensors allocated to specific water installations in the bathrooms. These external sensors may be directly mounted to water pipes in close proximity to the water installations and may provide valuable data to the apparatus. Preferably, these sensors use Bluetooth but other low power means may also be used. Specifically, one option is to apply these external sensors to a toilet in order to detect if the toilet is running with water. Also, an external sensor may be provided to or close to a water tap, such as a audio sensor, that may detect running water from the water tap. In combination, if both external sensors are provided, it may detect valuable information, such as identifying derived symptoms among user of the bathroom, including diseases such as incontinence and cystitis.

## Claims

1. An apparatus (100) for reducing or controlling water consumption in bathrooms, the apparatus (100) comprising a housing (105) and being configured to receive data from one or more water consumption locations in a bathroom, the housing (105) comprising:
one or more sensors (107) including a motion sensor (110) configured to detect a movement in a certain distance from the apparatus in a bathroom as a first indication of activity in the bathroom, an audio sensor (115) configured to monitor a sound of running water from one or more water consumption locations in a bathroom as a second indication of activity in the bathroom, optionally a humidity sensor (120) configured to monitor the ambient humidity in a bathroom as a third indication of activity in the bathroom, and optionally an ambient temperature sensor (200);
a controller (125) configured to qualify at least a part of the data received from the one or more sensors (107) by means of an algorithm to obtain qualified data, wherein the controller (125) is configured to qualify at least a part of the data received from the audio sensor (115) as qualified audio data by means of an algorithm based on a reference audio standard;
a transmitting member (130) configured to transmit the qualified data at a given point of time to an external unit (135), optionally via a mobile device (230), by means of low power signals; and
a unique identification means (140) being readable or scannable by a mobile device and configured to include output data based on the qualified data for reducing or controlling water consumption in the bathroom.

2. The apparatus according to claim 1, wherein the controller (125) is configured to qualify at least a part of the data received from the motion sensor (110) as qualified motion data by means of an algorithm based on a reference motion standard.

3. The apparatus according to claim 1 or 2, wherein the one or more sensors (107) includes a humidity sensor (120) configured to monitor the ambient humidity in a bathroom as a third indication of activity in the bathroom.

4. The apparatus according to claim 3, wherein the identification means (140) is configured to include output data if motion data and audio data at a given point of time are qualified by the controller (125), and humidity data is qualified by the controller (125) or the external unit (135) to encompass a predetermined change in humidity as the third indication of activity in the bathroom.

5. The apparatus according to any one of the preceding claims, wherein the one or more sensors (107) includes an ambient temperature sensor (200).

6. The apparatus according to any one of the preceding claims, wherein the one or more sensors (107) includes a carbon dioxide sensor, and the identification means (140) is configured to include output data if carbon dioxide data at a given point of time is qualified by the controller (125) or the external unit (135).

7. The apparatus according to any one of the preceding claims, wherein the housing (105) further comprises a commands receiving member (145) configured to receive commands based on the qualified data at a given point of time from the external unit (135) by means of low power signals, and the controller (125) is configured to integrate the commands.

8. The apparatus (100) according to any one of the preceding claims, wherein the housing (105) comprises:
a front cover (170) including a transparent centrally arranged portion (175) facing the front of said cover (170) that allows visibility to a display (180) positioned below the transparent centrally arranged portion (175), and a portion with a substantially thinner wall thickness (185) that allows functioning of the motion sensor (110) located below the level of the surface of the housing;
a back cover (190) connectable and sealable with the front cover (170), the back cover (190) encasing a printing board (195), the printing board (195) including the display (180) operable to visualize the unique matrix barcode (140), the one and more sensors, the controller (125), and the transmitting member (130);
a back plate (210) connectable and sealable with the back cover (190), the back plate and the back cover encasing a power supply member (220) for operating the apparatus.

9. The apparatus according to any of the preceding claims, wherein the output data includes one or more of water-saving tips, shower statistics, water tap statistics, toilet usage statistics, running water statistics and running water alerts.

10. A system (201) for reducing or controlling water consumption in bathrooms, the system comprising:
an apparatus (100) according to any one of the preceding claims;
an external unit (135) configured to receive qualified data from the apparatus (100) at a given point of time, optionally via a mobile device (230), by means of low power signals, and being configured to process data based on the qualified data and/or based on further qualification of data, such as humidity data, and configured to incorporate output data in a unique identification means (140) being readable by a mobile device (230) on the apparatus (100) for reducing or controlling water consumption in a bathroom;
optionally a commands receiving member (240) in the apparatus (100) configured to receive commands based on the qualified data at a given point of time from the external unit (135) by means of low power signals, and the controller (125) being configured to integrate the commands; and
a mobile device (230) operable to read the unique identification means (140).

11. The system according to claim 10, wherein the one or more external sensors (235) include one or more external temperature sensors mounted to a water inlet (240) of a local installation (250) in the bathroom, including one or more inlets of a toilet (255), a water tap (257) or a shower (260), and the one or more external temperature sensors being configured to detect a temperature difference of the water in the inlet (240) compared to ambient temperature over time, whereby running water may be detected.

12. The system according to any one of claims 10-11, wherein the one or more external sensors (235) includes an external temperature sensor mounted to a water inlet of a first local installation in the bathroom, such as a toilet (255), in combination with an external audio sensor in proximity to a water inlet of a second local installation in the bathroom and/or an external temperature sensor mounted to the water inlet of a second local installation in the bathroom, such as a water tap (257), whereby coordinated usage of both installations may be detected.

13. The system according to any one of claims 10-12, further including an alert for running water to the mobile device (230) and/or to the external unit (135).

14. A method of reducing or controlling water consumption in bathrooms, the method comprising the steps of:
providing an apparatus (100) being configured to receive data from one or more water consumption locations in a bathroom, the apparatus comprising a housing (105) including one or more sensors (107), a controller (125), a transmitting member (130), and a unique identification means (140);
detecting a movement in a certain distance from the apparatus (100) in a bathroom as a first indication of activity in the bathroom by means of a motion sensor (110), detecting a sound of running water from one or more water consumption locations in a bathroom as a second indication of activity in the bathroom by means of an audio sensor (115), optionally detecting the ambient humidity in a bathroom as a third indication of activity in the bathroom by means of a humidity sensor (120), and optionally detecting ambient temperature by means of an ambient temperature sensor (200);
qualifying at least a part of the data received from the one or more sensors (107) by means of an algorithm to obtain qualified data, wherein the data includes data received from the audio sensor (115) as qualified audio data based on a reference audio standard;
transmitting the qualified data at a given point of time to an external unit (135), optionally via a mobile device (230), by means of low power signals; and
subjecting output data based on the qualified data for reducing or controlling water consumption in the bathroom to a unique identification means (140) being readable or scannable by a mobile device (230).

15. The method according to claim 14, wherein the method is applied in an apparatus (100) and/or a system (201) according to claims 1-13.

## Patentansprüche

1. Einrichtung (100) zum Reduzieren oder Steuern des Wasserverbrauchs in Badezimmern, wobei die Einrichtung (100) ein Gehäuse (105) umfasst und konfiguriert ist, um Daten von einem oder mehreren Wasserverbrauchsstellen in einem Badezimmer zu empfangen, wobei das Gehäuse (105) umfasst:
einen oder mehrere Sensoren (107), der/die einen Bewegungssensor (110), der konfiguriert ist, um eine Bewegung in einer bestimmten Entfernung von der Einrichtung in einem Badezimmer als ersten Hinweis auf Aktivität im Badezimmer zu erkennen, einen Audiosensor (115), der konfiguriert ist, um ein Geräusch von fließendem Wasser von einem oder mehreren Wasserverbrauchsstandstellen in einem Badezimmer als zweiten Hinweis auf Aktivität im Badezimmer zu überwachen, optional einen Feuchtigkeitssensor (120), der konfiguriert ist, um die Umgebungsfeuchtigkeit in einem Badezimmer als dritten Hinweis auf Aktivität im Badezimmer zu überwachen, und optional einen Umgebungstemperatursensor (200) beinhaltet/beinhalten;
eine Steuereinheit (125), die konfiguriert ist, um zumindest einen Teil der von dem einen oder mehreren Sensoren (107) empfangenen Daten anhand eines Algorithmus zu qualifizieren, um qualifizierte Daten zu erhalten, wobei die Steuereinheit (125) konfiguriert ist, um zumindest einen Teil der von dem Audiosensor (115) empfangenen Daten anhand eines auf einem Referenz-Audiostandard basierenden Algorithmus als qualifizierte Audiodaten zu qualifizieren;
ein Sendeelement (130), das konfiguriert ist, um die qualifizierten Daten zu einem gegebenen Zeitpunkt anhand von Signalen mit geringer Leistung, optional über eine mobile Vorrichtung (230), an eine externe Einheit (135) zu senden; und
ein eindeutiges Identifikationsmittel (140), das durch eine mobile Vorrichtung lesbar oder scanbar ist und konfiguriert ist, um Ausgabedaten basierend auf den qualifizierten Daten zum Reduzieren oder Steuern des Wasserverbrauchs im Badezimmer zu beinhalten.

2. Einrichtung nach Anspruch 1, wobei die Steuereinheit (125) konfiguriert ist, um zumindest einen Teil der vom Bewegungssensor (110) empfangenen Daten anhand eines auf einem Referenzbewegungsstandard basierenden Algorithmus als qualifizierte Bewegungsdaten zu qualifizieren.

3. Einrichtung nach Anspruch 1 oder 2, wobei der eine oder mehrere Sensoren (107) einen Feuchtigkeitssensor (120) beinhaltet/beinhalten, der/die konfiguriert ist/sind, um die Umgebungsfeuchtigkeit in einem Badezimmer als dritten Hinweis auf Aktivität im Badezimmer zu überwachen.

4. Einrichtung nach Anspruch 3, wobei das Identifikationsmittel (140) konfiguriert ist, um Ausgabedaten zu beinhalten, wenn Bewegungsdaten und Audiodaten zu einem gegebenen Zeitpunkt durch die Steuereinheit (125) qualifiziert werden, und Feuchtigkeitsdaten durch die Steuereinheit (125) oder die externe Einheit (135) qualifiziert werden, eine vorbestimmte Änderung der Feuchtigkeit als dritten Hinweis auf Aktivität im Badezimmer einzuschließen.

5. Einrichtung einem der vorstehenden Ansprüche, wobei der eine oder mehrere Sensoren (107) einen Umgebungstemperatursensor (200) beinhaltet/beinhalten.

6. Einrichtung nach einem der vorstehenden Ansprüche, wobei der eine oder mehrere Sensoren (107) einen Kohlendioxidsensor beinhaltet/beinhalten und das Identifikationsmittel (140) konfiguriert ist, um Ausgabedaten zu beinhalten, wenn Kohlendioxiddaten zu einem gegebenen Zeitpunkt durch die Steuereinheit (125) oder die externe Einheit (135) qualifiziert werden.

7. Einrichtung nach einem der vorstehenden Ansprüche, wobei das Gehäuse (105) weiter ein Befehlsempfangselement (145) umfasst, das konfiguriert ist, um zu einem gegebenen Zeitpunkt auf den qualifizierten Daten basierende Befehle von der externen Einheit (135) anhand von Signalen mit geringer Leistung zu empfangen, und wobei die Steuereinheit (125) konfiguriert ist, um die Befehle zu integrieren.

8. Einrichtung (100) nach einem der vorstehenden Ansprüche, wobei das Gehäuse (105) umfasst:
eine vordere Abdeckung (170), die einen transparenten, mittig angeordneten Abschnitt (175) beinhaltet, der zur Vorderseite der Abdeckung (170) weist und die Sicht auf eine Anzeige (180) erlaubt, die unterhalb des transparenten, mittig angeordneten Abschnitts (175) positioniert ist, und einen Abschnitt mit einer wesentlich dünneren Wandstärke (185), der die Funktion des Bewegungssensors (110) erlaubt, der sich unterhalb der Ebene der Oberfläche des Gehäuses befindet;
eine hintere Abdeckung (190), die mit der vorderen Abdeckung (170) verbindbar und versiegelbar ist, wobei die hintere Abdeckung (190) eine Druckplatte (195) umschließt und die Druckplatte (195) die Anzeige (180) beinhaltet, die betreibbar ist, um den eindeutigen Matrix-Barcode (140), den einen und mehrere Sensoren, die Steuereinheit (125) und das Sendeelement (130) zu visualisieren;
eine hintere Platte (210), die mit der hinteren Abdeckung (190) verbindbar und versiegelbar ist, wobei die hintere Platte und die hintere Abdeckung ein Stromversorgungselement (220) zum Betreiben der Einrichtung umschließen.

9. Einrichtung nach einem der vorstehenden Ansprüche, wobei die Ausgabedaten eines oder mehr von Tipps zum Wassersparen, Duschstatistiken, Wasserhahnstatistiken, Toilettenbenutzungsstatistiken, Statistiken zu fließendem Wasser und Warnungen zu fließendem Wasser beinhalten.

10. System (201) zum Reduzieren oder Steuern des Wasserverbrauchs in Badezimmern, wobei das System umfasst:
eine Einrichtung (100) nach einem der vorstehenden Ansprüche;
eine externe Einheit (135), die konfiguriert ist, um zu einem gegebenen Zeitpunkt qualifizierte Daten von der Einrichtung (100) anhand von Signalen mit geringer Leistung, optional über eine mobile Vorrichtung (230) zu empfangen, und die konfiguriert ist, um Daten basierend auf den qualifizierten Daten und/oder basierend auf einer weiteren Qualifizierung von Daten, wie etwa Feuchtigkeitsdaten zu verarbeiten, und konfiguriert, um Ausgabedaten in ein eindeutiges Identifikationsmittel (140) zu integrieren, das von einer mobilen Vorrichtung (230) in der Einrichtung (100) zum Reduzieren oder Steuern des Wasserverbrauchs in einem Badezimmer lesbar ist;
optional ein Befehlsempfangselement (240) in der Einrichtung (100), das konfiguriert ist, um zu einem gegebenen Zeitpunkt auf den qualifizierten Daten basierende Befehle von der externen Einheit (135) anhand von Signalen mit geringer Leistung zu empfangen, und die Steuereinheit (125) konfiguriert ist, um die Befehle zu integrieren; und
eine mobile Vorrichtung (230), die betreibbar ist, um das eindeutige Identifikationsmittel (140) zu lesen.

11. System nach Anspruch 10, wobei der eine oder mehrere externe Sensoren (235) einen oder mehrere externe Temperatursensoren beinhaltet/beinhalten, die an einem Wassereinlass (240) einer lokalen Installation (250) im Badezimmer montiert sind, die einen oder mehrere Einlässen einer Toilette (255), eines Wasserhahns (257) oder einer Dusche (260) beinhalten, und wobei der eine oder mehrere externe Temperatursensoren konfiguriert ist/sind, um einen Temperaturunterschied des Wassers im Einlass (240) im Vergleich zur Umgebungstemperatur im Laufe der Zeit zu erkennen, wodurch fließendes Wasser erkannt werden kann.

12. System nach einem der Ansprüche 10-11, wobei der eine oder mehrere externe Sensoren (235) einen externen Temperatursensor beinhaltet/beinhalten, der an einem Wassereinlass einer ersten lokalen Installation im Badezimmer, wie beispielsweise einer Toilette (255), montiert ist, in Kombination mit einem externen Audiosensor in der Nähe eines Wassereinlasses einer zweiten lokalen Installation im Badezimmer und/oder ein externer Temperatursensor, der am Wassereinlass einer zweiten lokalen Installation im Badezimmer, beispielsweise einem Wasserhahn (257), montiert ist, wodurch eine koordinierte Nutzung beider Installationen erkannt werden kann.

13. System nach einem der Ansprüche 10-12, das weiter eine Warnung für fließendes Wasser an die mobile Vorrichtung (230) und/oder an die Außeneinheit (135) beinhaltet.

14. Verfahren zum Reduzieren oder Steuern des Wasserverbrauchs in Badezimmern, wobei das Verfahren die Schritte umfasst zum:
Bereitstellen einer Einrichtung (100), die konfiguriert ist, um Daten von einem oder mehreren Wasserverbrauchsstellen in einem Badezimmer zu empfangen, wobei die Einrichtung ein Gehäuse (105) umfasst, das eine oder mehrere Sensoren (107), eine Steuereinheit (125), ein Sendeelement (130) und ein eindeutiges Identifikationsmittel (140) beinhaltet;
Erkennen einer Bewegung in einer bestimmten Entfernung von der Einrichtung (100) in einem Badezimmer als ersten Hinweis auf Aktivität im Badezimmer anhand eines Bewegungssensors (110), Erkennen eines Geräusches von fließendem Wasser von einem oder mehreren Wasserverbrauchsstellen in einem Badezimmer als zweiten Hinweis auf Aktivität im Badezimmer anhand eines Audiosensors (115), optional Erkennen der Umgebungsfeuchtigkeit in einem Badezimmer als dritten Hinweis auf Aktivität im Badezimmer anhand eines Feuchtigkeitssensors (120), und optional Erkennen der Umgebungstemperatur anhand eines Umgebungstemperatursensors (200);
Qualifizieren von zumindest einem Teil der von einem oder mehreren Sensoren (107) empfangenen Daten anhand eines Algorithmus, um qualifizierte Daten zu erhalten, wobei die Daten Daten beinhalten, die von dem Audiosensor (115) als qualifizierte Audiodaten basierend auf einem Referenz-Audiostandard empfangen wurden;
Senden der qualifizierten Daten zu einem gegebenen Zeitpunkt, optional über eine mobile Vorrichtung (230), an eine externe Einheit (135) anhand von Signalen mit geringer Leistung; und
Unterwerfen von auf den qualifizierten Daten basierenden Ausgabedaten zum Reduzieren oder Steuern des Wasserverbrauchs im Badezimmer einem eindeutigen Identifikationsmittel (140), das durch eine mobile Vorrichtung (230) lesbar oder scanbar ist.

15. Verfahren nach Anspruch 14, wobei das Verfahren in einer Einrichtung (100) und/oder einem System (201) nach den Ansprüchen 1-13 angewendet wird.

## Revendications

1. Appareil (100) pour réduire ou réguler la consommation d'eau dans des salles de bains, l'appareil (100) comprenant un boîtier (105) et étant configuré pour recevoir des données provenant d'un ou de plusieurs emplacements de consommation d'eau dans une salle de bains, le boîtier (105) comprenant :
un ou plusieurs capteurs (107) incluant un capteur de mouvement (110) configuré pour détecter un mouvement à une certaine distance de l'appareil dans une salle de bains en tant que première indication d'activité dans la salle de bains, un capteur audio (115) configuré pour surveiller un son d'eau courante provenant d'un ou de plusieurs emplacements de consommation d'eau dans une salle de bains en tant que deuxième indication d'activité dans la salle de bains, facultativement un capteur d'humidité (120) configuré pour surveiller l'humidité ambiante dans une salle de bains en tant que troisième indication d'activité dans la salle de bains, et facultativement un capteur de température ambiante (200) ;
un dispositif de commande (125) configuré pour qualifier au moins une partie des données reçues des un ou plusieurs capteurs (107) au moyen d'un algorithme pour obtenir des données qualifiées, dans lequel le dispositif de commande (125) est configuré pour qualifier au moins une partie des données reçues du capteur audio (115) en tant que données audio qualifiées au moyen d'un algorithme basé sur une norme audio de référence ;
un élément de transmission (130) configuré pour transmettre les données qualifiées à un instant donné à une unité externe (135), facultativement par le biais d'un dispositif mobile (230), au moyen de signaux de faible puissance ; et
un moyen d'identification unique (140) pouvant être lu ou scanné par un dispositif mobile et configuré pour inclure des données de sortie basées sur les données qualifiées pour réduire ou réguler la consommation d'eau dans la salle de bains.

2. Appareil selon la revendication 1, dans lequel le dispositif de commande (125) est configuré pour qualifier au moins une partie des données reçues du capteur de mouvement (110) en tant que données de mouvement qualifiées au moyen d'un algorithme basé sur une norme de mouvement de référence.

3. Appareil selon la revendication 1 ou 2, dans lequel les un ou plusieurs capteurs (107) incluent un capteur d'humidité (120) configuré pour surveiller l'humidité ambiante dans une salle de bains en tant que troisième indication d'activité dans la salle de bains.

4. Appareil selon la revendication 3, dans lequel le moyen d'identification (140) est configuré pour inclure des données de sortie si des données de mouvement et des données audio à un instant donné sont qualifiées par le dispositif de commande (125), et si des données d'humidité sont qualifiées par le dispositif de commande (125) ou l'unité externe (135) pour englober un changement prédéterminé d'humidité en tant que troisième indication d'activité dans la salle de bains.

5. Appareil selon l'une quelconque des revendications précédentes, dans lequel les un ou plusieurs capteurs (107) incluent un capteur de température ambiante (200).

6. Appareil selon l'une quelconque des revendications précédentes, dans lequel les un ou plusieurs capteurs (107) incluent un capteur de dioxyde de carbone et le moyen d'identification (140) est configuré pour inclure des données de sortie si des données de dioxyde de carbone à un instant donné sont qualifiées par le dispositif de commande (125) ou l'unité externe (135).

7. Appareil selon l'une quelconque des revendications précédentes, dans lequel le boîtier (105) comprend en outre un élément de réception de commandes (145) configuré pour recevoir des commandes basées sur les données qualifiées à un instant donné en provenance de l'unité externe (135) au moyen de signaux de faible puissance, et le dispositif de commande (125) est configuré pour intégrer les commandes.

8. Appareil (100) selon l'une quelconque des revendications précédentes, dans lequel le boîtier (105) comprend :
un couvercle avant (170) incluant une partie transparente agencée centralement (175) faisant face à l'avant dudit couvercle (170) qui permet une visibilité sur un affichage (180) positionné sous la partie transparente agencée centralement (175), et une partie avec une épaisseur de paroi sensiblement plus mince (185) qui permet le fonctionnement du capteur de mouvement (110) situé sous le niveau de la surface du boîtier ;
un couvercle arrière (190) pouvant être relié et scellé au couvercle avant (170), le couvercle arrière (190) renfermant une carte d'impression (195), la carte d'impression (195) incluant l'affichage (180) pouvant être utilisé pour visualiser le code-barres matriciel unique (140), les un ou plusieurs capteurs, le dispositif de commande (125) et l'élément de transmission (130) ;
une plaque arrière (210) pouvant être reliée et scellée au couvercle arrière (190), la plaque arrière et le couvercle arrière renfermant un élément d'alimentation électrique (220) pour faire fonctionner l'appareil.

9. Appareil selon l'une quelconque des revendications précédentes, dans lequel les données de sortie incluent un ou plusieurs de conseils d'économie d'eau, de statistiques de douche, de statistiques de robinet d'eau, de statistiques d'utilisation des toilettes, de statistiques d'eau courante et d'alertes d'eau courante.

10. Système (201) pour réduire ou réguler la consommation d'eau dans des salles de bains, le système comprenant :
un appareil (100) selon l'une quelconque des revendications précédentes,
une unité externe (135) configurée pour recevoir des données qualifiées en provenance de l'appareil (100) à un instant donné, facultativement par le biais d'un dispositif mobile (230), au moyen de signaux de faible puissance, et étant configurée pour traiter des données sur la base des données qualifiées et/ou sur la base d'une qualification supplémentaire de données, telles que des données d'humidité, et configurée pour incorporer des données de sortie dans un moyen d'identification unique (140) qui est lisible par un dispositif mobile (230) sur l'appareil (100) pour réduire ou réguler la consommation d'eau dans une salle de bains ;
facultativement un élément de réception de commandes (240) dans l'appareil (100) configuré pour recevoir des commandes basées sur les données qualifiées à un instant donné en provenance de l'unité externe (135) au moyen de signaux de faible puissance, et le dispositif de commande (125) étant configuré pour intégrer les commandes ; et
un dispositif mobile (230) pouvant être utilisé pour lire le moyen d'identification unique (140).

11. Système selon la revendication 10, dans lequel les un ou plusieurs capteurs externes (235) incluent un ou plusieurs capteurs de température externes montés sur une entrée d'eau (240) d'une installation locale (250) dans la salle de bains, incluant une ou plusieurs entrées d'une toilette (255), d'un robinet d'eau (257) ou d'une douche (260), et les un ou plusieurs capteurs de température externes étant configurés pour détecter une différence de température de l'eau dans l'entrée (240) par rapport à la température ambiante au fil du temps, selon lequel de l'eau courante peut être détectée.

12. Système selon l'une quelconque des revendications 10-11, dans lequel les un ou plusieurs capteurs externes (235) incluent un capteur de température externe monté sur une entrée d'eau d'une première installation locale dans la salle de bains, telle qu'une toilette (255), en combinaison avec un capteur audio externe à proximité d'une entrée d'eau d'une seconde installation locale dans la salle de bains et/ou un capteur de température externe monté sur l'entrée d'eau d'une seconde installation locale dans la salle de bains, telle qu'un robinet d'eau (257), selon lequel une utilisation coordonnée des deux installations peut être détectée.

13. Système selon l'une quelconque des revendications 10-12, incluant en outre une alerte pour de l'eau courante envoyée au dispositif mobile (230) et/ou à l'unité externe (135).

14. Procédé de réduction ou de régulation de la consommation d'eau dans des salles de bains, le procédé comprenant les étapes consistant à :
fournir un appareil (100) qui est configuré pour recevoir des données en provenance d'un ou de plusieurs emplacements de consommation d'eau dans une salle de bains, l'appareil comprenant un boîtier (105) incluant un ou plusieurs capteurs (107), un dispositif de commande (125), un élément de transmission (130) et un moyen d'identification unique (140) ;
détecter un mouvement à une certaine distance de l'appareil (100) dans une salle de bains en tant que première indication d'activité dans la salle de bains au moyen d'un capteur de mouvement (110), détecter un bruit d'eau courante provenant d'un ou de plusieurs emplacements de consommation d'eau dans une salle de bains en tant que deuxième indication d'activité dans la salle de bains au moyen d'un capteur audio (115), facultativement détecter l'humidité ambiante dans une salle de bains en tant que troisième indication d'activité dans la salle de bains au moyen d'un capteur d'humidité (120), et facultativement détecter la température ambiante au moyen d'un capteur de température ambiante (200) ;
qualifier au moins une partie des données reçues d'un ou de plusieurs capteurs (107) au moyen d'un algorithme pour obtenir des données qualifiées, dans lequel les données incluent des données reçues du capteur audio (115) en tant que données audio qualifiées sur la base d'une norme audio de référence ;
transmettre les données qualifiées à un instant donné à une unité externe (135), facultativement par le biais d'un dispositif mobile (230), au moyen de signaux de faible puissance ; et
soumettre des données de sortie basées sur les données qualifiées pour réduire ou réguler la consommation d'eau dans la salle de bains à un moyen d'identification unique (140) qui peut être lu ou scanné par un dispositif mobile (230).

15. Procédé selon la revendication 14, dans lequel le procédé est appliqué dans un appareil (100) et/ou un système (201) selon les revendications 1-13.
